(19) Europäisches Patentamt
European Patent Office
Office européen des brevets

(11) Veröffentlichungsnummer: **0 525 571 A1**

# (12) EUROPÄISCHE PATENTANMELDUNG

(21) Anmeldenummer: **92112359.2**

(22) Anmeldetag: **20.07.92**

(51) Int. Cl.$^5$: **C07D 215/14**, A61K 31/47

(30) Priorität: **31.07.91 DE 4125270**

(43) Veröffentlichungstag der Anmeldung:
**03.02.93 Patentblatt 93/05**

(84) Benannte Vertragsstaaten:
**AT BE CH DE DK ES FR GB GR IT LI LU NL PT SE**

(71) Anmelder: **BAYER AG**

**W-5090 Leverkusen 1 Bayerwerk(DE)**

(72) Erfinder: **Matzke, Michael, Dr.**
**Am Ringelbusch 15**
**W-5600 Wuppertal 1(DE)**
Erfinder: **Mohrs, Klaus-Helmut, Dr.**
**Wildsteig 24**
**W-5600 Wuppertal 1(DE)**
Erfinder: **Raddatz, Siegfried, Dr.**
**Jakob-Böhme-Strasse 21**
**W-5000 Köln 80(DE)**
Erfinder: **Fruchtmann, Romanis**
**Konrad-Adenauer-Ufer 79**
**W-5000 Köln 1(DE)**
Erfinder: **Hatzelmann, Armin, Dr.**
**Nagelsbaum 44**
**W-5093 Burscheid(DE)**
Erfinder: **Kohlsdorfer, Christian, Dr.**
**Franz-Stryck-Strasse 16**
**W-5042 Erfstadt(DE)**
Erfinder: **Müller-Peddinghaus, Reiner, Prof. Dr.**
**Klutstein 22a**
**W-5060 Bergisch Gladbach(DE)**
Erfinder: **Theisen-Popp, Pia, Dr.**
**Horbacher Strasse 368**
**W-5100 Aachen(DE)**

## (54) Chinolin-2-yl-methoxybenzylhydroxyharnstoffe als Lipoxygenasehemmer.

(57) Die neuen Chinolin-2-yl-methoxybenzylhydroxyharnstoffe können durch Umsetzung von entsprechenden Ketonen mit Hydroxylamin und Reduktion der Ketoxime sowie anschließende Reaktion mit Isocyanaten hergestellt werden.

Die neuen Stoffe können als Wirkstoffe in Arzneimitteln eingesetzt werden.

EP 0 525 571 A1

Die Erfindung betrifft Chinolin-2-yl-methoxybenzylhydroxyharnstoffe, deren Derivate, Verfahren zu ihrer Herstellung sowie ihre Verwendung in Arzneimitteln.

Es ist bereits bekannt, daß Indol-, Benzofuran- und Benzothiophenhydroxyharnstoffeund deren Derivate eine lipoxygenaseeinhibierende Wirkung besitzen [vgl. EP 416 609 A2]. Außerdem werden in der Publikation EP 388 429 (PCT WO89/04299) Furan- und Pyrroloxim-hydroxyharnstoffe beschrieben.

Die vorliegende Erfindung betrifft nun Chinolin-2-yl-methoxybenzylhydroxyharnstoffe der allgemeinen Formel (I)

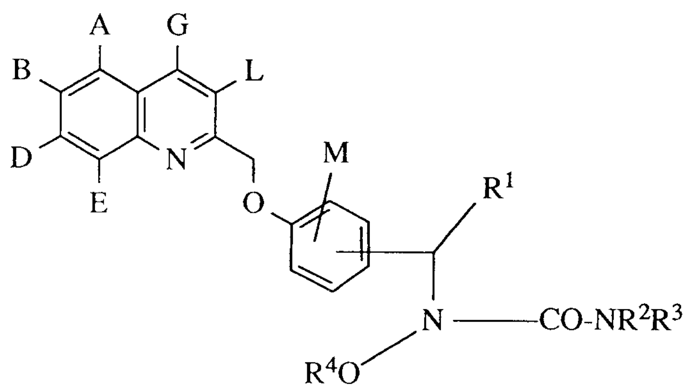

(I)

in welcher

A, B, D, E, G, L und M gleich oder verschieden sind und
für Wasserstoff, Hydroxy, Halogen, Cyano, Carboxy, Nitro, Trifluormethyl, Trifluormethoxy oder
für geradkettiges oder verzweigtes Alkyl oder Alkoxy mit jeweils bis zu 8 Kohlenstoffatomen stehen, oder
für Aryl mit 6 bis 10 Kohlenstoffatomen stehen, das gegebenenfalls durch Halogen, Hydroxy, Nitro oder Cyano substituiert ist,

$R^1$ für Cycloalkyl oder-alkenyl mit 3 bis 12 Kohlenstoffatomen steht, für geradkettiges oder verzweigtes Alkyl mit bis zu 8 Kohlenstoffatomen steht,

$R^2$ und $R^3$ gleich oder verschieden sind und
für Wasserstoff, geradkettiges oder verzweigtes Alkyl mit bis zu 8 Kohlenstoffatomen oder
für Phenyl oder Benzyl stehen, oder

$R^2$ für Wasserstoff steht

und

$R^3$ für eine Gruppe der Formel $-SO_2R^5$ steht,
worin

$R^5$ geradkettiges oder verzweigtes Alkyl mit bis zu 8 Kohlenstoffatomen bedeutet, das gegebenenfalls durch Halogen, Nitro, Cyano, Hydroxy, Trifluormethyl oder durch Aryl mit 6 bis 10 Kohlenstoffatomen substituiert ist,
oder Aryl mit 6 bis 10 Kohlenstoffatomen bedeutet, das bis zu 3-fach gleich oder verschieden durch Halogen, Nitro, Cyano, Hydroxy, geradkettiges oder verzweigtes Alkyl, Alkylthio oder Alkoxy mit bis zu 8 Kohlenstoffatomen, Trifluormethyl oder Trifluormethoxy substituiert ist,

$R^4$ für Wasserstoff oder für geradkettiges oder verzweigtes Acyl mit bis zu 8 Kohlenstoffatomen oder Benzoyl steht,

und deren Salze.

Im Rahmen der vorliegenden Erfindung werden physiologisch unbedenkliche Salze bevorzugt. Physiologisch unbedenkliche Salze der Chinolin-2-yl-methoxybenzylhydroxyharnstoffe und deren Derivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung können außerdem Salze von Metallen bevorzugt von einwertigen Metallen, wie Alkalimetalle, und Ammoniumsalze sein. Bevorzugt werden Natrium-, Kalium- und

Ammoniumsalze.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher

A, B, D, E, G, L und M gleich oder verschieden sind und für Wasserstoff, Hydroxy, Fluor, Chlor, Brom, Carboxy, Nitro, Trifluormethyl, Trifluormethoxy oder für geradkettiges oder verzweigtes Alkyl oder Alkoxy mit jeweils bis zu 6 Kohlenstoffatomen stehen, oder für Phenyl stehen, das gegebenenfalls durch Fluor, Chlor, Brom, Hydroxy, Nitro oder Cyano substituiert ist,

$R^1$ für Cyclobutyl, Cyclopentyl, Cyclohexyl, Cycloheptyl oder Cyclooctyl steht für geradkettiges oder verzweigtes Alkyl mit bis zu 6 Kohlenstoffatomen steht,

$R^2$ und $R^3$ gleich oder verschieden sind und für Wasserstoff oder für geradkettiges oder verzweigtes Alkyl mit bis zu 6 Kohlenstoffatomen stehen,

oder

$R^2$ für Wasserstoff steht

und

$R^3$ für eine Gruppe der Formel $-SO_2R^5$ steht,
worin

$R^5$ geradkettiges oder verzweigtes Alkyl mit bis zu 6 Kohlenstoffatomen bedeutet, das gegebenenfalls durch Fluor, Chlor, Nitro, Cyano, Hydroxy oder durch Phenyl substituiert ist, oder Phenyl bedeutet, das gegebenenfalls bis zu 2-fach gleich oder verschieden durch Fluor, Chlor, Brom, Nitro, Cyano, Hydroxy, geradkettiges oder verzweigtes Alkyl, Alkylthio oder Alkoxy mit bis zu 6 Kohlenstoffatomen, Trifluormethyl oder Trifluormethoxy substituiert ist,

$R^4$ für Wasserstoff oder für geradkettiges oder verzweigtes Acyl mit bis zu 6 Kohlenstoffatomen oder Benzoyl steht

und deren Salze.

Besonders bevorzugt sind Verbindungen der allgemeinen Formel (I),
in welcher

A, B, D, E, G, L und M gleich oder verschieden sind und für Wasserstoff, Hydroxy, Fluor, Chlor, Brom oder geradkettiges oder verzweigtes Alkyl mit bis zu 4 Kohlenstoffatomen stehen,

$R^1$ für Cyclopentyl, Cyclohexyl oder Cycloheptyl steht, für geradkettiges oder verzweigtes Alkyl mit bis zu 4 Kohlenstoffatomen steht,

$R^2$ und $R^3$ gleich oder verschieden sind und für Wasserstoff oder geradkettiges oder verzweigtes Alkyl mit bis zu 4 Kohlenstoffatomen stehen, oder

$R^2$ für Wasserstoff steht,

und

$R^3$ für eine Gruppe der Formel $-SO_2R^5$ steht,
worin

$R^5$ geradkettiges oder verzweigtes Alkyl mit bis zu 4 Kohlenstoffatomen bedeutet, das gegebenenfalls durch Fluor, Chlor oder Phenyl substituiert ist, oder Phenyl bedeutet, das gegebenenfalls durch Fluor, Chlor, Brom oder durch geradkettiges oder verzweigtes Alkyl mit bis zu 4 Kohlenstoffatomen substituiert ist,

$R^4$ für Wasserstoff oder für geradkettiges oder verzweigtes Acyl mit bis zu 4 Kohlenstoffatomen oder Benzoyl steht

und deren Salze.

Ganz besonders bevorzugt sind Verbindungen der allgemeinen Formel (I),
in welcher

A, B, D, E, G, L und M für Wasserstoff stehen. Ebenso sind Verbindungen ganz besonders bevorzugt, in denen der Rest $-HC(R^1)N(OR^4)(CO-NR^2R^3)$ in 4-Stellung zum Chinolylmethoxyrest steht.

Außerdem wurde ein Verfahren zur Herstellung der erfindungsgemäßen Verbindungen der allgemeinen Formel (I) gefunden, das dadurch gekennzeichnet ist, daß man Verbindungen der allgemeinen Formel (II)

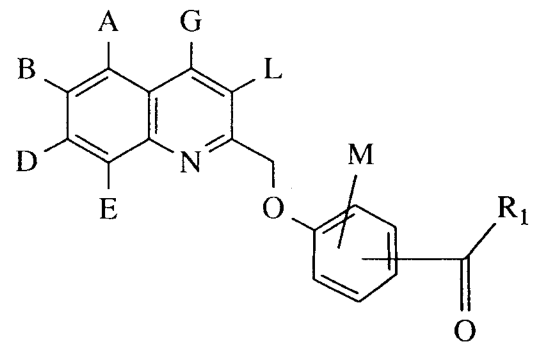

(II)

in welcher
A, B, D, E, G, L, M und $R^1$ die oben angegebene Bedeutung haben,
zunächst in inerten Lösemitteln, gegebenenfalls in Anwesenheit einer Base mit Hydroxylaminhydrochlorid zu den Ketoximen der allgemeinen Formel (III)

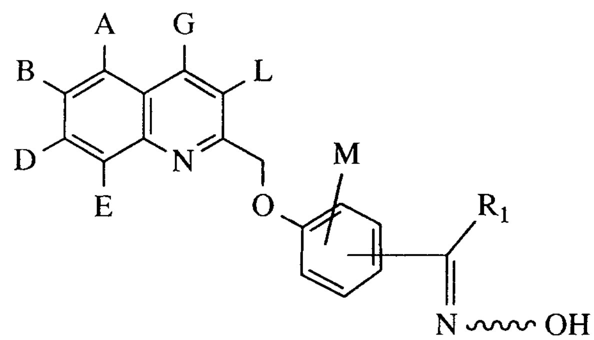

(III)

in welcher
A, B, D, E, G, L, M und $R^1$ die oben angegebene Bedeutung haben,
umsetzt, anschließend in inerten Lösemitteln, gegebenenfalls in Anwesenheit von Säuren, mit Reduktionsmitteln in die Hydroxylamine der allgemeinen Formel (IV)

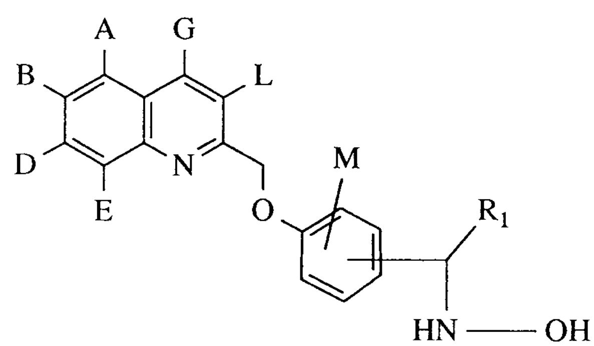

(IV)

in welcher
A, B, D, E, G, L, M und $R^1$ die oben angegebene Bedeutung haben,
überführt, und in einem letzten Schritt, im Fall, daß $R^2$ und $R^3$ = H mit ($C_1$-$C_6$)-Trialkylsilylisocyanaten, vorzugsweise mit Trimethylsilylisocyanat, und im Fall, daß $R^2$ = H und $R^3 \neq$ H mit Isocyanaten der allgemeinen Formel (V)

T-N = C = O (V)

in welcher

T für $(C_1-C_8)$-Alkyl, Phenyl oder für den Rest $-SO_2R^5$ steht, worin

$R^5$ die oben angegebene Bedeutung hat,

umsetzt,

oder

Hydroxylamine der allgemeinen Formel (IV) zunächst mit gasförmiger HCl und anschließend mit Phosgen in die Verbindungen der allgemeinen Formel (VI)

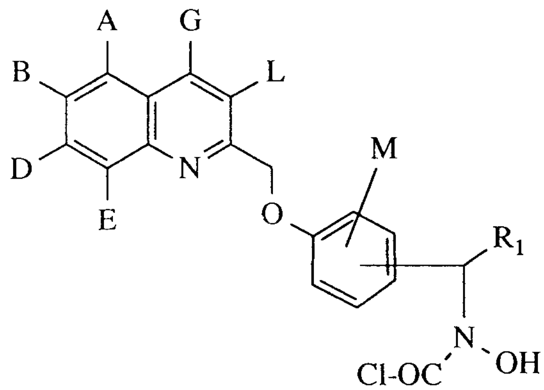

(VI)

in welcher

A, B, D, E, G, L, M und $R^1$ die oben angegebene Bedeutung haben,

in inerten Lösemitteln überführt und nachfolgend, gegebenenfalls in inerten Lösemitteln, mit Aminen der allgemeinen Formel (VII)

$HNR^2R^3$ (VII)

in welcher

$R^2$ und $R^3$ gleich oder verschieden sind und die oben angegebene Bedeutung haben,

umsetzt und im Fall, daß $R^4 \neq H$, Verbindungen der allgemeinen Formel (Ia)

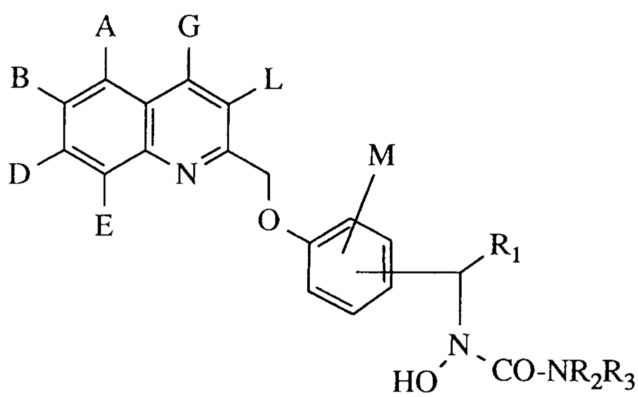

(Ia)

in welcher

A, B, D, E, G, L, M, $R^1$, $R^2$ und $R^3$ die oben angegebene Bedeutung haben,

mit üblichen Acylierungsmitteln, vorzugsweise mit Säurehalogeniden der allgemeinen Formel (VIII)

$R^{4'}$-CO-X (VIII)

in welcher

$R^{4'}$ die oben angegebene Bedeutung von $R^4$ hat, aber nicht für Wasserstoff steht

und

X für eine in Acylierungsmitteln übliche "leaving group", vorzugsweise für Chlor steht,

in inerten Lösemitteln, gegebenenfalls in Anwesenheit einer Base, acyliert.

Das erfindungsgemäße Verfahren kann durch folgendes Formelschema beispielhaft erläutert werden:

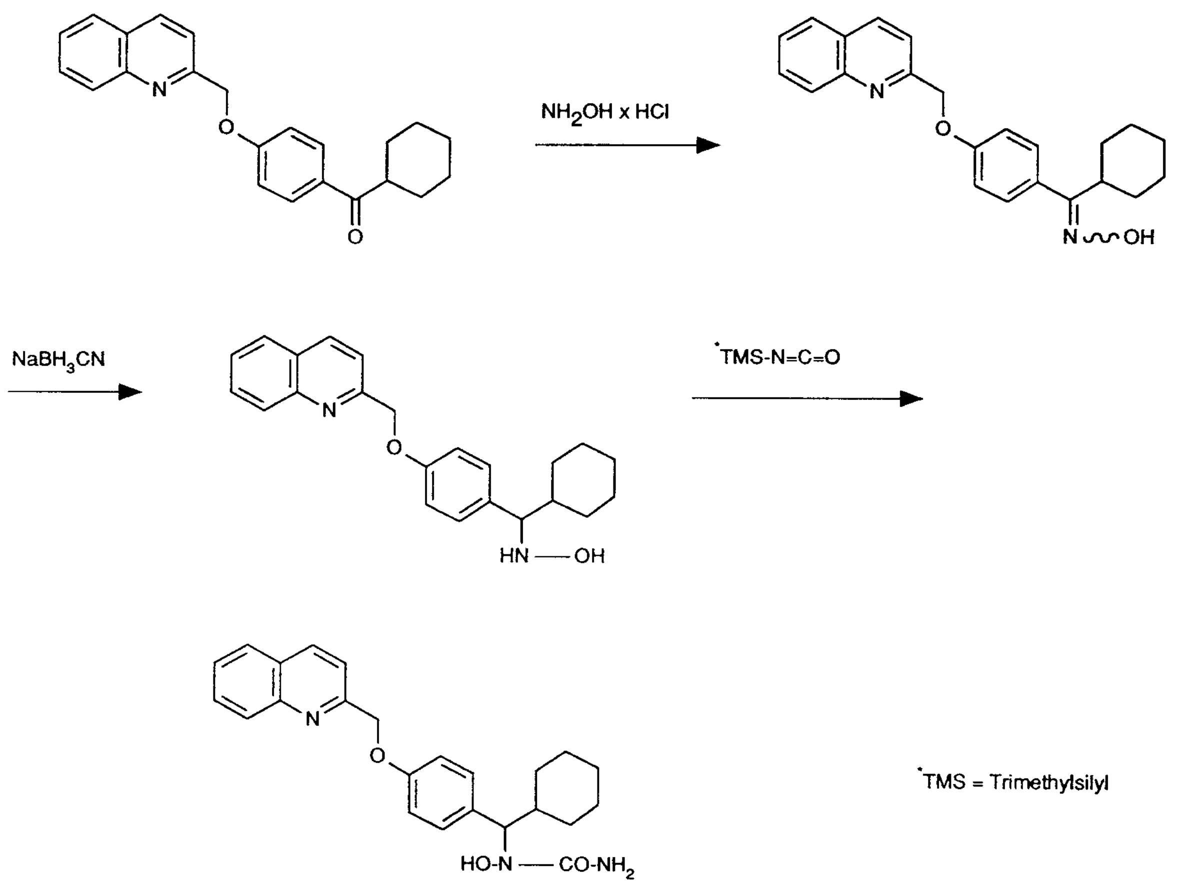


Als Lösemittel für die Umsetzung mit Hydroxylaminhydrochlorid eignen sich die üblichen organischen Lösemittel, die sich unter den Reaktionsbedingungen nicht verändern. Hierzu gehören bevorzugt Alkohole wie Methanol, Ethanol, Propanol oder Isopropanol oder Kohlenwasserstoffe wie Benzol, Toluol, Xylol, Hexan, Cyclohexan oder Erdölfraktionen, oder Acetonitril, Dimethylsulfoxid oder Dimethylformamid. Bevorzugt sind Methanol und Ethanol.

Als Basen eignen sich organische Amine (Trialkyl($C_1$-$C_6$)amine), wie beispielsweise Triethylamin oder Heterocyclen wie Pyridin, Methylpiperidin, Piperidin oder Morpholin. Bevorzugt ist Pyridin.

Die Base wird in einer Menge von 1 mol bis 20 mol, bevorzugt von 5 mol bis 10 mol, bezogen auf 1 mol der Verbindung der allgemeinen Formel (II) eingesetzt.

Die Reaktion erfolgt in einem Temperaturbereich von 0 °C bis +80 °C, bevorzugt bei Raumtemperatur.

Für die Reduktion der Oxime der allgemeinen Formel (III) eignen sich im allgemeinen als Reduktionsmittel Hydride, wie komplexe Borhydride und Borhydrid-Amin-Komplexeoder Aluminiumhydride. Bevorzugt werden hierbei Natriumborhydrid, Natriumcyanoborhydrid oder Borpyridin-Komplexe eingesetzt. Besonders bevorzugt ist Natriumcyanoborhydrid.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden. Bevorzugt ist Methanol.

Die Reduktion mit Natriumcyanoborhydrid wird im allgemeinen in Gegenwart von Protonensäuren durchgeführt. Hierzu gehören bevorzugt anorganische Säuren wie beispielsweise Salzsäure oder Schwefelsäure, oder organische Carbonsäuren mit 1 bis 6 C-Atomen, gegebenenfalls substituiert durch Fluor, Chlor und/oder Brom, wie beispielsweise Essigsäure, Trifluoressigsäure, Trichloressigsäure oder Propionsäure,

oder Sulfonsäuren mit $C_1$-$C_4$-Alkylresten oder mit Arylresten wie beispielsweise Methansulfonsäure, Ethansulfonsäure, Benzolsulfonsäure oder Toluolsulfonsäure.

Als Säuren werden für alle anderen Verfahrensschritte im allgemeinen ebenfalls Mineralsäuren eingesetzt. Bevorzugt werden hierbei Chlorwasserstoffsäure, Bromwasserstoffsäure, Schwefelsäure, Phosphorsäure oder aber Gemische der genannten Säuren eingesetzt. Bevorzugt ist stets Chlorwasserstoffsäure.

Die Säuren werden in Abhängigkeit der jeweiligen Reaktanden mit der für die Einstellung eines pH-Wertes von 3 benötigten Menge zugegeben.

Die Reduktion erfolgt im allgemeinen in einem Temperaturbereich von 0 °C bis +60 °C, vorzugsweise bei Raumtemperatur.

Die Umsetzung wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Die Umsetzung der Verbindungen der allgemeinen Formel (IV) mit den Isocyanaten erfolgt in üblichen organischen Lösemitteln, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffen wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexymethylphosphorsäuretriamid, Acetonitril, Aceton oder Nitromethan. Ebenso ist es möglich, Gemische der genannten Lösemittel zu verwenden. Bevorzugt sind Dioxan und Methylenchlorid.

Die Reaktionen verlaufen im allgemeinen in einem Temperaturbereich von 0 °C bis +150 °C, vorzugsweise von +20 °C bis +120 °C.

Als Lösemittel für die Umsetzung mit den Isocyanaten eignen sich die oben aufgeführten Ether, Kohlenwasserstoffe oder Halogenkohlenwasserstoffe oder deren Gemische, bevorzugt Ether wie beispielsweise Diethylether oder Tetrahydrofuran, oder Halogenkohlenwasserstoffe wie beispielsweise Methylenchlorid oder Chloroform. Besonders bevorzugt ist Methylenchlorid.

Die Umsetzung mit Sulfonylisocyanaten erfolgt im allgemeinen in einem Temperaturbereich von -78 °C bis +120 °C, bevorzugt von -50 °C bis +100 °C.

Die Umsetzung mit den Isocyanaten wird im allgemeinen bei Normaldruck durchgeführt. Es ist aber auch möglich bei Überdruck oder bei Unterdruck zu arbeiten (z.B. in einem Bereich von 0,5 bis 5 bar).

Im allgemeinen setzt man 1 bis 3 Mol, bevorzugt 1 bis 2 Mol, besonders bevorzugt 1 Mol Isocyanat, bezogen auf 1 Mol der Verbindung der allgemeinen Formel (IV) ein.

Die Alkyl-, Phenyl- und Silylisocyanate der allgemeinen Formel (V) sind bekannt oder können nach üblicher Methode hergestellt werden [vgl. z.B. Beilstein 4 (3), 1861; Beilstein 12, 437].

Die Sulfonylisocyanate (VI) sind literaturbekannt [vgl. C. King, J. Org. Chem. 25, 352 (1960); F. Effenberger, R. Gleiter, Chem. Ber. 97, 1576 (1964); H. Ulrich, A.A.R. Sayigh, Angew. Chem. 78, 761 (1966); Houben-Weyl VIII, 128].

Die Umsetzung der Verbindungen der allgemeinen Formel (VI) mit Ammoniak ($R^2$/$R^3$ = H), Ammoniumchlorid oder den Aminen der allgemeinen Formel (VII) erfolgt entweder in einem der oben angegebenen Lösemittel oder mit der jeweiligen Aminkomponente als Lösemittel, vorzugsweise mit dem jeweiligen Amin im Überschuß, in einem Temperaturbereich von -10 °C bis +50 °C und bei Normaldruck.

Die Verbindungen der allgemeinen Formel (VI) sind neu und können nach dem oben aufgeführten Verfahren hergestellt werden.

Die Amine der allgemeinen Formel (VII) sind bekannt [vgl. Houben-Weyl's "Methoden der organischen Chemie", Bd. XI/1 und XI/2].

Als "leaving group" in den Acylierungsmitteln der allgemeinen Formel (VIII) sind im allgemeinen Reste wie beispielsweise Chlor, Brom, Thiazolyl, Methansulfonyloxy oder Aryloxy gemeint. Bevorzugt ist Chlor. Die Acylierungsmittel sind literaturbekannt.

Die Acylierung verläuft im allgemeinen in einem Temperaturbereich von 0 °C bis +120 °C, vorzugsweise bei +30 °C bis +90 °C, in einem der oben aufgeführten Lösemittel, vorzugsweise in Pyridin und bei Normaldruck.

Als Basen für die Acylierung eignen sich anorganische oder organische Basen. Hierzu gehören vorzugsweise Alkalihydroxide wie z.B. Natriumhydroxid oder Kaliumhydroxid, Erdalkalihydroxide wie z.B. Bariumhydroxid, Alkalicarbonate wie Natriumcarbonat oder Kaliumcarbonat, Erdalkalicarbonate wie Calciumcarbonat, oder organische Amine (Trialkyl($C_1$-$C_6$)amine) wie Triethylamin, oder Heterocyclen wie Pyridin, Methylpiperidin, Piperidin oder Morpholin. Bevorzugt ist Triethylamin.

Die Verbindungen der allgemeinen Formel (Ia) sind ebenfalls neu und können nach dem oben aufgeführten Verfahren hergestellt werden.

Die Verbindungen der allgemeinen Formel (II) sind teilweise bekannt oder im Fall, daß $R^1$ für Cycloalkyl

steht neu und können beispielsweise hergestellt werden,
indem man Verbindungen der allgemeinen Formel (IX)

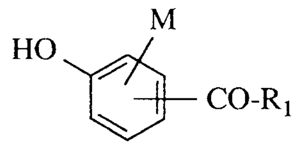

(IX)

in welcher
M und $R^1$ die oben angegebene Bedeutung haben,
in inerten Lösemitteln, gegebenenfalls in Anwesenheit einer Base, mit Verbindungen der allgemeinen Formel (X),

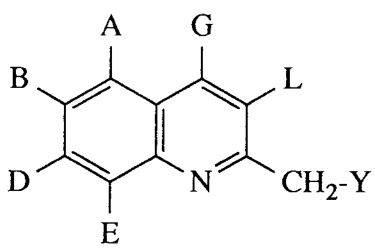

(X)

in welcher
A, B, D, E, G und L die oben angegebene Bedeutung haben
und
Y für Halogen, vorzugsweise für Chlor oder Brom steht,
verethert.

Die Veretherung kann in inerten organischen Lösemitteln, gegebenenfalls in Anwesenheit einer Base durchgeführt werden. Lösemittel für die Veretherung können inerte organische Lösemittel sein, die sich unter den Reaktionsbedingungen nicht verändern. Hierzu gehören vorzugsweise Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische der Lösemittel einzusetzen.

Als Basen für die Veretherung können anorganische oder organische Basen eingesetzt werden. Hierzu gehören vorzugsweise Alkalihydroxide wie z.B. Natriumhydroxid oder Kaliumhydroxid, Erdalkalihydroxide wie z.B. Bariumhydroxid, Alkalicarbonate wie Natriumcarbonat oder Kaliumcarbonat, Erdalkalicarbonate wie Calciumcarbonat, oder organische Amine (Trialkyl($C_1$-$C_6$)amine) wie Triethylamin, oder Heterocyclen wie Pyridin, Methylpiperidin, Piperidin oder Morpholin.

Es ist auch möglich, als Basen Alkalimetalle wie Natrium und deren Hydride, wie Natriumhydrid, einzusetzen.

Die Veretherung erfolgt im allgemeinen in einem Temperaturbereich von 0°C bis +150°C, bevorzugt von +10°C bis +100°C.

Die Veretherung wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 Mol Halogenid, bezogen auf 1 Mol der Verbindung der allgemeinen Formel (IX) ein. Die Base wird im allgemeinen in einer Menge von 0,5 bis 5 Mol, bevorzugt von 1 bis 3 Mol bezogen auf das Halogenid (X) eingesetzt.

Die Verbindungen der allgemeinen Formeln (IX) und (X) sind an sich bekannt oder können nach üblicher Methode hergestellt werden [vgl. CAS 38 459-58-4 und Chem. Ber. 120, 649 (1987)].

Die Verbindungen der allgemeinen Formeln (III) und (IV) sind größtenteils neu (insbesondere wenn $R^1$ für Cycloalkyl steht) und können nach dem oben aufgeführten Verfahren hergestellt werden.

Die erfindungsgemäßen Chinolin-2-yl-methoxybenzylhydroxyharnstoffe und deren Derivate können als Wirkstoffe in Arzneimitteln eingesetzt werden. Die Stoffe können als Hemmer von enzymatischen Reaktionen im Rahmen des Arachidonsäurestoffwechsels, insbesondere der Lipoxygenase, wirken.

Sie sind somit bevorzugt zur Behandlung und Verhütung von Erkrankungen der Atemwege wie

Allergien/Asthma, Bronchitis, Emphysem, Schocklunge, pulmonaler Hypertonie, Entzündungen/Rheuma und Ödemen, Thrombosen und Thromboembolien, Ischämien (periphere, cardiale, cerebrale Durchblutungsstörungen), Herz- und Hirninfarkten, Angina pectoris, Arteriosklerose, bei Gewebstransplatalionen, Dermatosen wie Psoriasis, entzündliche Dermatosen und zur Cytoprotection im Gastrointestinaltrakt geeignet.

Die erfindungsgemäßen Chinolin-2-yl-methoxybenzylhydroxyharnstoffe und deren Derivate können sowohl in der Humanmedizin als auch in der Veterinärmedizin angewendet werden.

Die pharmakologischen Wirkungen der erfindungsgemäßen Substanzen werden durch folgende Methode bestimmt:

Als Maß für die Lipoxyenase-Hemmung wurde die Freisetzung von Leukotrien $B_4$ ($LTB_4$) an polymorphkernigen Humanleukozyten (PMN) nach Zugabe von Substanzen und Ca-Ionophor mittels reverse phase HPLC nach Borgeat, P. et al., Proc. Nat. Acad. Sci., 76, 2148 - 2152 (1979), bestimmt.

In der Tabelle 1 sind beispielhaft die nach diesem Test erzielten Werte einiger erfindungsgemäßer Verbindungen aufgeführt:

Tabelle 1

| Beispiel-Nr. | 5-LO $IC_{50}$ (µmol/l) |
|---|---|
| 1 | 0,25 |
| 2 | 1,2 |

Zur vorliegenden Erfindung gehören auch pharmazeutische Zubereitungen, die neben inerten, nichttoxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Ausgansverbindungen

Beispiel I

4-Methoxyphenylcyclohexylketon

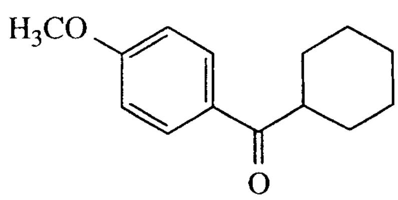

Zu einer Suspension von 44,4 g (0,33 mol) Aluminiumchlorid in 120 ml Dichlorethan werden bei Raumtemperatur 42,6 g (0,291 mol) Cyclohexancarbonsäurechlorid und anschließend 30 g (0,277 mol) Anisol getropft. Die Reaktionsmischung wird 1 h gerührt und über Nacht stehen gelassen. Zur Aufarbeitung

wird der Ansatz in Eiswasser gegossen und die wäßrige Phase mit Dichlormethan extrahiert. Die organische Phase wird mit 1 N NaOH und Wasser gewaschen, über Natriumsulfat getrocknet und im Vakuum eingeengt.
Ausbeute: 58,6 g (97% der Theorie)
Fp.: 59-62 ° C

Beispiel II

4-Hydroxyphenylcyclohexylketon

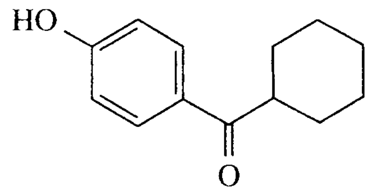

Eine Lösung von 11 g (50,4 mmol) 4-Methoxyphenylcyclohexylketon in 730 ml Dichlormethan p.a. wird bei -70 ° C unter Argon mit 101 ml einer 1 M Lösung von Bortribromid (101 mmol) in Dichlormethan versetzt und anschließend 18 h bei Raumtemperatur belassen. Es wird im Vakuum eingeengt, der Rückstand mit 500 ml Ethanol und 80 ml Wasser aufgenommen und 3 h unter Rückfluß erhitzt. Nach dem Einengen im Vakuum wird in Dichlormethan aufgenommen, die organische Phase mit Wasser gewaschen, über Natriumsulfat getrocknet und im Vakuum zur Trockene eingeengt. Das Rohprodukt wird an Kieselgel mit Dichlormethan/Methanol (20:1) chromatographiert.
Ausbeute: 7,9 g (76,7% der Theorie)
Fp.: 81-84 ° C

Beispiel III

4-(Chinolin-2-yl-methoxy)phenylcyclohexylketon

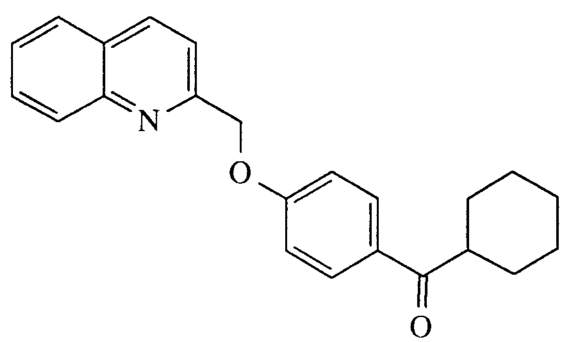

7,85 g (38,4 mmol) 4-Hydroxyphenylcyclohexylketon werden nach Zugabe von 12 g (86,8 mmol) Kaliumcarbonat und 200 ml Dimethylformamid 2 h unter Rückfluß erhitzt. Anschließend werden bei Raumtemperatur 8,54 g (39,9 mmol) 2-Chlormethylchinolinhydrochlorid zugegeben. Der Reaktionsansatz wird 7 h bei 100 ° C gerührt und nachfolgend im Vakuum eingeengt. Der Rückstand wird in Dichlormethan aufgenommen, die organische Phase mit Wasser gewaschen, über Natriumsulfat getrocknet und im Vakuum eingeengt. Es folgt eine säulenchromatographische Reinigung an Kieselgel mit Dichlormethan/Methanol (50:1).
Ausbeute: 12,3 g (92,7% der Theorie)
Fp.: 103-104 ° C

Beispiel IV

4-(Chinolin-2-yl-methoxy)phenylcyclohexylketoxim

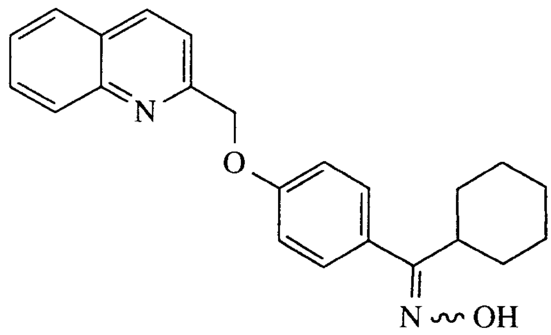

Eine Lösung von 15,9 g (46 mmol) 4-(Chinolin-2-yl-methoxy)phenylcyclohexylketon in 100 ml Ethanol und 33 ml Pyridin wird nach Zugabe von 4,77 g (68,6 mmol) Hydroxylamin Hydrochlorid 18 h bei Raumtemperatur gerührt. Zur Aufarbeitung wird im Vakuum eingeengt und der Rückstand in Dichlormethan aufgenommen. Die organische Phase wird mit Wasser gewaschen, über Natriumsulfat getrocknet und im Vakuum zur Trockene eingeengt. Ausbeute: 11,4g (68,8% der Theorie) farblose Kristalle Fp.: 167-171 ° C

Beispiel V

N-[4-(Chinolin-2-yl-methoxy)phenyl-cyclohexyl]methyl-hydroxylamin

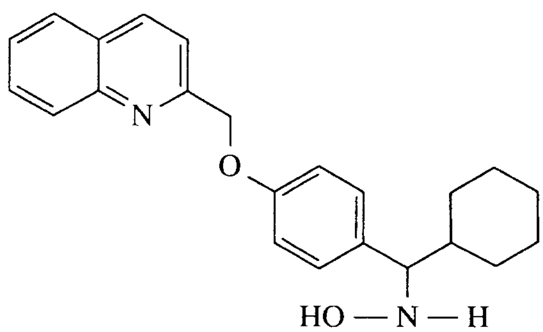

Ein Gemisch von 7 g (19,4 mmol) 4-(Chinolin-2-yl-methoxy)phenylcyclohexylketoxim und 860 mg (13,7 mmol) Nathriumcyanoborhydrid in 50 ml Methanol p.a. wird mit insgesamt 28 ml 2 M methanolischer HCl auf pH 3 eingestellt und bei Raumtemperatur gerührt. Nach 3 h werden nochmals 430 mg (6,84 mmol) Natriumcyanoborhydrid zugegeben. Nach weiteren 2 h wird der Reaktionsansatz eingeengt, mit Wasser und 6 N KOH bis pH 9 versetzt und mit Dichlormethan extrahiert. Die organische Phase wird über Natriumsulfat getrocknet und im Vakuum eingeengt. Das Rohprodukt wird säulenchromatographisch mit Dichlormethan/Methanol (20:1) gereinigt und aus Diethylether kristallisiert.
Ausbeute: 3,9 g (55,5% der Theorie), farblose Kristalle
Fp.: 101-102 ° C

Beispiel VI

4-Methoxyphenylcyclopentylketon

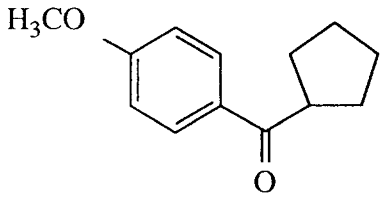

In Analogie zur Vorschrift des Beispiels I wird die Titelverbindung aus 31 g (0,287 mol) Anisol, 46 g (0,345 mol) Aluminiumchlorid und 40 g (0,302 mol) Cyclopentancarbonsäurechlorid hergestellt.
Ausbeute: 52,2 g (89 % der Theorie), Öl

Beispiel VII

4-Hydroxyphenylcyclopentylketon

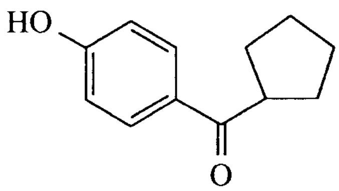

Die Titelverbindung wird in Analogie zur Vorschrift des Beispiels II aus 30 g (0,147 mol) der Verbindung aus Beispiel VI und 290 ml einer 1 M Lösung von Bortribromid (0,29 mmol) in Dichlormethan dargestellt.
Ausbeute: 21,95 g (78,6 % der Theorie), Öl

Beispiel VIII

4-(Chinolin-2-yl-methoxy)phenylcyclopentylketon

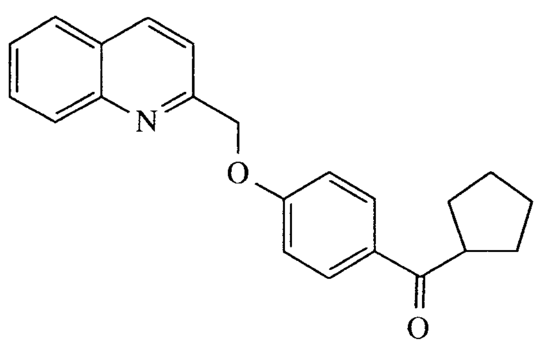

In Analogie zur Vorschrift des Beispiels III wird die Titelverbindung aus 21,5 g (0,113 mol) der Verbindung aus Beispiel VII, 25 g (0,116 mol) 2-Chlormethylchinolinhydrochlorid und 35,15 g (0,254mol) Kaliumcarbonat hergestellt.
Ausbeute: 23 g (61,4 % der Theorie), Öl

Beispiel IX

4-(Chinolin-2-yl-methoxy)phenylcyclopentylketoxim

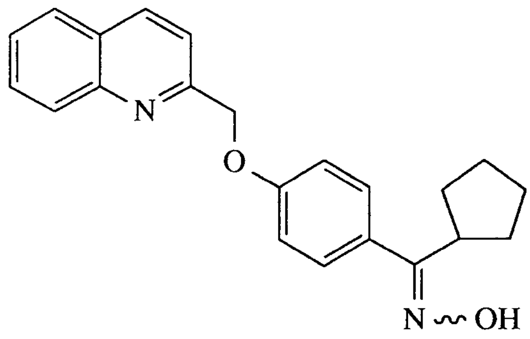

In Analogie zur Vorschrift des Beispiels IV wird die Titelverbindung aus 20 g (60,3 mmol) der Verbindung aus Beispiel VIII und 6,3 g (90,7 mmol) Hydroxylamin Hydrochlorid hergestellt.
Ausbeute: 10,6 g (50,7 % der Theorie)
Fp.: 174-176 ° C

Beispiel X

N-[4-(Chinolin-2-yl-methoxy)phenyl-cyclopentyl]methylhydroxylamin

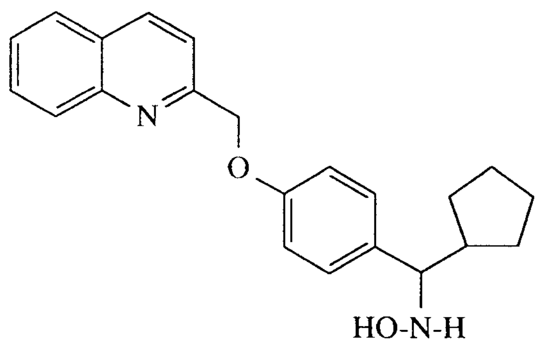

Die Titelverbindung wird in Analogie zur Vorschrift des Beispiels V aus 6,15 g (17,8 mmol) der Verbindung aus Beispiel IX und 1,18 g (18,8 mmol) Natriumcyanoborhydrid hergestellt.
Ausbeute: 3,0g (48,5 % der Theorie)
Fp.: 116-117 ° C

Beispiel XI

4-Methoxyphenylcycloheptylketon

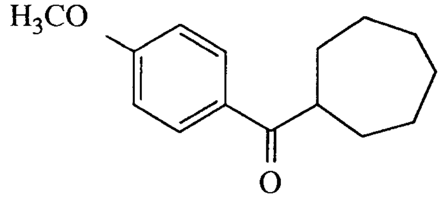

In Analogie zur Vorschrift des Beispiels I wird die Titelverbindung aus 31,7 g (0,293 mol) Anisol, 47 g (0,352 mol) Aluminiumchlorid und 49,5 g (0,308 mol) Cycloheptancarbonsäurechlorid hergestellt.
Ausbeute: 64,2 g (94,3 % der Theorie), Öl

Beispiel XII

4-Hydroxyphenylcycloheptylketon

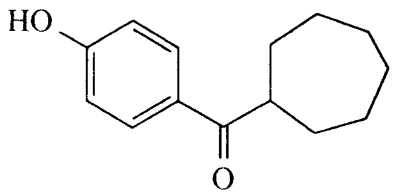

Die Titelverbindung wird in Analogie zur Vorschrift des Beispiels II aus 53,2 g (0,229 mol) der Verbindung aus Beispiel XI und 458 ml einer 1 M Lösung von Bortribromid (0,458 mol) in Dichlormethan dargestellt.
Ausbeute: 34g (68 % der Theorie), Öl

Beispiel XIII

4-(Chinolin-2-yl-methoxy)phenylcycloheptylketon

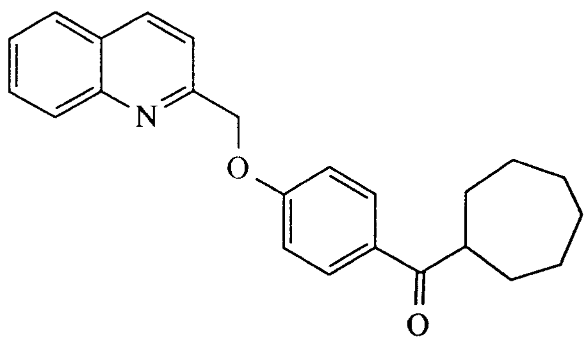

In Analogie zur Vorschrift des Beispiels III wird die Titelverbindung aus 10 g (45,8 mmol) der Verbindung aus Beispiel XII, 10,3 g (48,1 mmol) 2-Chlormethylchinolinhydrochlorid und 13,8 g (0,10 mol) Kaliumcarbonat hergestellt.
Ausbeute: 13,1 g (79,5 % der Theorie)

Beispiel XIV

4-(Chinolin-2-yl-methoxy)phenylcycloheptylketoxim

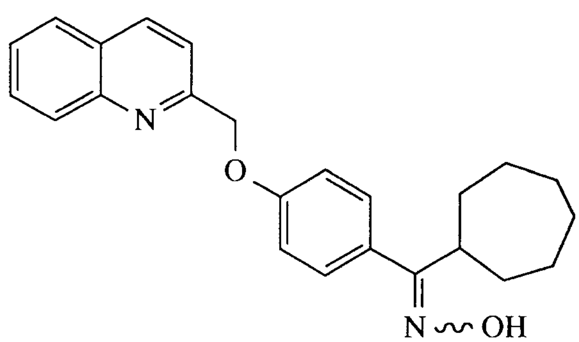

In Analogie zur Vorschrift des Beispiels IV wird die Titelverbindung aus 12,9 g (35,9 mmol) der Verbindung aus Beispiel XIII und 3,75 g (53,9 mmol) Hydroxylamin Hydrochlorid hergestellt.
Ausbeute: 8,7 g (65 %)
Fp.: 185-186 ° C

Beispiel XV

N-[4-(Chinolin-2-yl-methoxy)phenyl-cycloheptyl]methylhydroxylamin

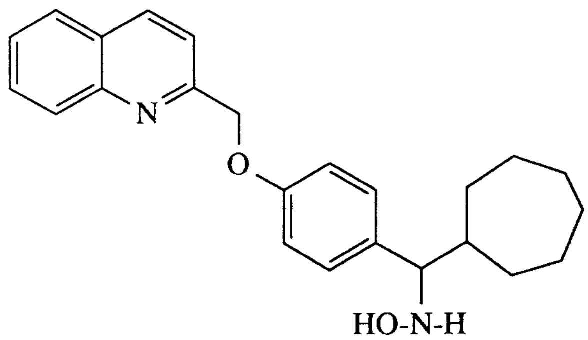

In Analogie zur Vorschrift des Beispiels V wird die Titelverbindung aus 8,5 g (22,7 mmol) der Verbindung aus Beispiel XIV und 1,5 g (23,9 mmol) Natriumcyanoborhydrid hergestellt.
Ausbeute: 2,6 g (30,4 % der Theorie), amorph

Beispiel XVI

( + )-N-[4-(Chinolin-2-yl-methoxy)phenyl-cyclohexyl]methyl-hydroxylamin, (1S)-(-)-Camphansäuresalz

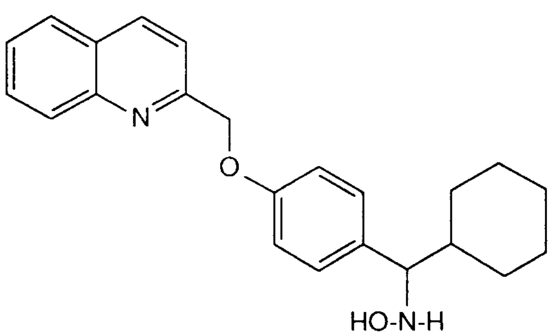

x

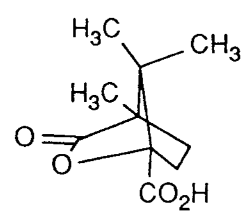

Zu einer Lösung aus 9,5 g (0,026 mol) der Verbindung aus Beispiel V in 26 ml Methanol p.a. wird eine Lösung aus 5,25 g (0,026 mol) (1S)-(-)-Camphansäure in 16 ml Methanol p.a. in der Siedehitze gegeben. Nach dem Abkühlen auf Raumtemperatur wird die Titelverbindung nach Zugabe von wenig Petrolether kristallisiert. Das Rohprodukt wird aus 22 ml Methanol p.a umkristallisiert.
Ausbeute: 2,6 g (17,6% d. Theorie), farblose Kristalle
Fp.: 123-125 ° C
$[\alpha]^{20}_{D}$ = +6,5 ° (c = 0,8 in DMSO)

Beispiel XVII

( + )-N-[4-(Chinolin-2-yl-methoxy)phenyl-cyclohexyl]methyl-hydroxylamin

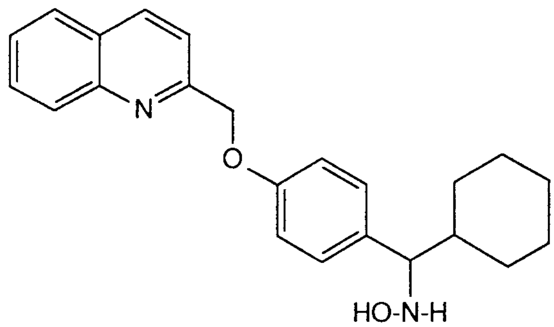

Verfahren A

Aus der racemischen Verbindung des Beispiels V wird die enantiomerenreine Titelverbindung durch präparative HPLC an Chiracel AD (Daicel) (Elutionsmittel: Petrolether /2-Propanol / Wasser / Trifluoressigsäure, 75:25:0,25:0,05) erhalten.

Verfahren B

Zur Freisetzung der Titelverbindung werden 2,6 g (4,64 mol) der Verbindung aus Beispiel XVI mit 20 ml 2 N NaOH und 40 ml Dichlormethan versetzt. Die Mischung wird bis zur klaren Phasentrennung geschüttelt. Die organische Phase wird eingeengt. Der Rückstand wird aus Diethylether umkristallisiert. Ausbeute: 1,5 g (93,7% der Theorie), farblose Kristalle
Fp.: 100-101 °C
Enantiomerenüberschuß: >99 %
$[\alpha]^{20}_D$ = +10,5 ° (c =0,97 in DMSO)
In Analogie zu den oben aufgeführten Herstellungsvorschriften können die in Tabelle 1 aufgeführten Verbindungen dargestellt werden:

Tabelle 1:

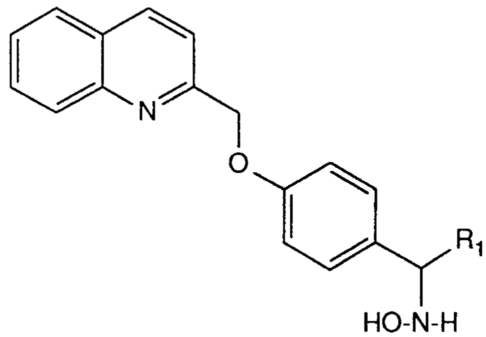

| Bsp.-Nr. | $R^1$ | Enantiomer |
|---|---|---|
| XVIII | 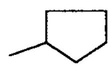 | (+) |
| XIX | 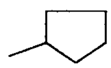 | (-) |
| XX | 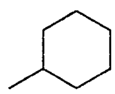 | (-) |
| XXI | 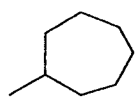 | (+) |
| XXII | 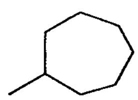 | (-) |

Herstellungsbeispiele

Beispiel 1

N-[4-(Chinolin-2-yl-methoxy)phenyl-cyclohexyl]methyl-N-hydroxyharnstoff

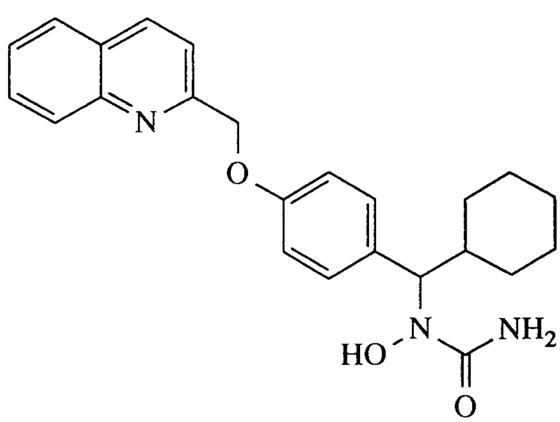

Zu einer Lösung von 2,7 g (7,45 mmol) N-[4-(Chinolin-2-yl-methoxy)phenyl-cyclohexyl]methyl-hydroxylamin in 25 ml Dioxan p.a. werden unter Argonatmosphäre bei Raumtemperatur 0,94 g (8,13 mmol) Trimethylsilylisocyanat getropft. Der Reaktionsansatz wird 2,5 h unter Argonatmosphäre auf 100 °C erhitzt, auf Raumtemperatur abgekühlt und mit gesättigter Ammoniumchloridlösung versetzt. Es wird mehrfach mit Ether extrahiert, die organische Phase über Natriumsulfat getrocknet und im Vakuum eingeengt. Das Produkt wird nach Säulenchromatographie an Kieselgel mit Dichlormethan/Methanol (9:1) erhalten und aus Ethanol kristallisiert.
Ausbeute: 1,3 g (43,0% der Theorie) farblose Kristalle
Fp.: 180-181 °C

Beispiel 2

N-[4-(Chinolin-2-yl-methoxy)phenyl-cyclopentyl]methyl-N-hydroxyharnstoff

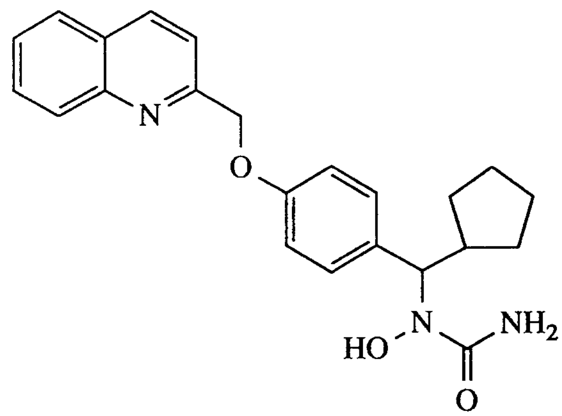

Zu einer Lösung von 3,35 g (9,61 mmol) N-[4-(Chinolin-2-yl-methoxy)phenylcycloheptyl]methyl-hydroxylamin in 70 ml Methylenchlorid p.a. werden unter Argonatmosphäre bei Raumtemperatur 1,22 g (10,6 mmol) Trimethylsilylisocyanat getropft. Der Reaktionsansatz wird 2 h bei Raumtemperatur gerührt und im Vakuum eingeengt. Das Produkt wird säulenchromatographisch an Kieselgel mit Dichlormethan/Methanol (20:1) gereinigt.
Ausbeute: 2,8 g (74,4 % der Theorie) farblose Kristalle
Fp.: 165-166 °C (Zersetzung)

Beispiel 3

N-[4-(Chinolin-2-yl-methoxy)phenyl-cycloheptyl]methyl-N-hydroxyharnstoff

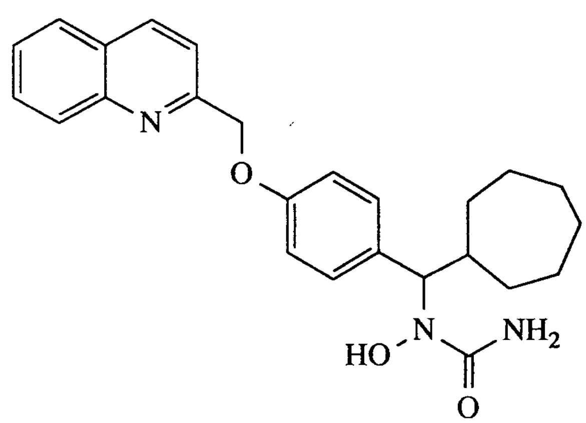

In Analogie zur Vorschrift des Beispiels 2 wird die Titelverbindung aus 2,3 g (6,11 mmol) der Verbindung aus Beispiel XV und 0,77 g (6,68 mol) Trimethylsilylisocyanat hergestellt.
Ausbeute: 1,55 g (56 % der Theorie) farblose Kristalle
Fp.: 138-140 ° C (Zersetzung)

Beispiel 4

(+)-N-[4-(Chinolin-2-yl-methoxy)phenyl-cyclohexyl]methyl-N-hydroxyharnstoff

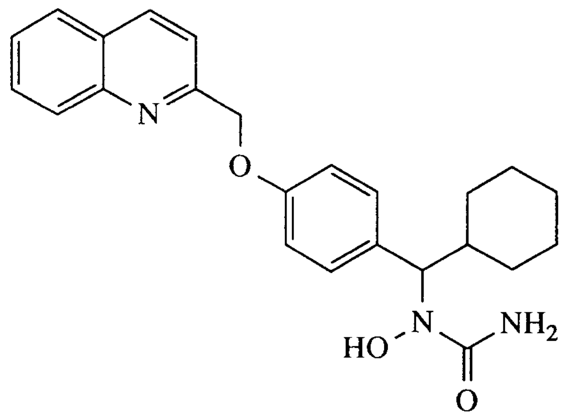

Zu einer Lösung aus 1,5 g (4,1 mmol) der Verbindung aus Beispiel XVII in 15 ml Dichlormethan p.a. werden bei Raumtemperatur 1,3 ml (10,2 mmol) Trimethylsilylisocyanat getropft. Der Reaktionsansatz wird 2 h bei Raumtemperatur gerührt. Anschließend wird mit Wasser gewaschen, die organische Phase über Natriumsulfat getrocknet und im Vakuum eingeengt. Das Rohprodukt wird aus Ethanol umkristallisiert.
Ausbeute: 1,0 g (59,5% der Theorie), farblose Kristalle
Fp.: 190-192 ° C
Enantiomerenüberschuß: >99%
$[\alpha]^{20}_{D}$ = +38,8 ° (c = 0,87 in DMSO)
In Analogie zu deroben aufgeführten Herstellungsvorschrift können die in Tabelle 2 aufgeführten Verbindungen dargestellt werden:

Tabelle 2:

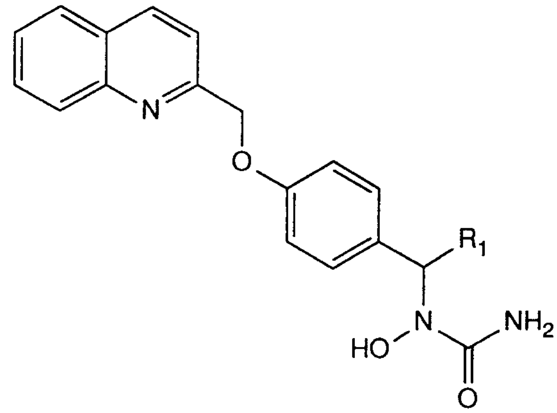

| Bsp.-Nr. | $R^1$ | Enantiomer |
|---|---|---|
| 5 | 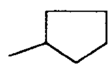 | (+) |
| 6 | 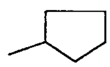 | (-) |
| 7 | 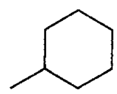 | (-) |
| 8 | 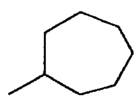 | (+) |
| 9 | 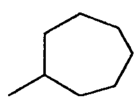 | (-) |

## Patentansprüche

**1.** Chinolin-2-yl-methoxybenzylhydroxyharnstoffe der allgemeinen Formel

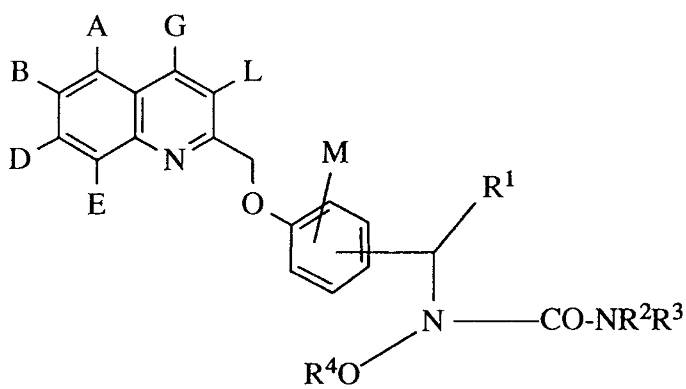

(I)

in welcher

A, B, D, E, G, L und M gleich oder verschieden sind und für Wasserstoff, Hydroxy, Halogen, Cyano, Carboxy, Nitro, Trifluormethyl, Trifluormethoxy oder für geradkettiges oder verzweigtes Alkyl oder Alkoxy mit jeweils bis zu 8

Kohlenstoffatomen stehen, oder
für Aryl mit 6 bis 10 Kohlenstoffatomen stehen, das gegebenenfalls durch Halogen, Hydroxy, Nitro oder Cyano substituiert ist.

$R^1$ für Cycloalkyl oder -alkenyl mit 3 bis 12 Kohlenstoffatomen steht, für geradkettiges oder verzweigtes Alkyl mit bis zu 8 Kohlenstoffatomen steht,

$R^2$ und $R^3$ gleich oder verschieden sind und
für Wasserstoff, geradkettiges oder verzweigtes Alkyl mit bis zu 8 Kohlenstoffatomen oder
für Phenyl oder Benzyl stehen, oder

$R^2$ für Wasserstoff steht

und

$R^3$ für eine Gruppe der Formel $-SO_2R^5$ steht,

worin

$R^5$ geradkettiges oder verzweigtes Alkyl mit bis zu 8 Kohlenstoffatomen bedeutet, das gegebenenfalls durch Halogen, Nitro, Cyano, Hydroxy, Trifluormethyl oder durch Aryl mit 6 bis 10 Kohlenstoffatomen substituiert ist,

oder
Aryl mit 6 bis 10 Kohlenstoffatomen bedeutet, das bis zu 3-fach gleich oder verschieden durch Halogen, Nitro, Cyano, Hydroxy, geradkettiges oder verzweigtes Alkyl, Alkylthio oder Alkoxy mit bis zu 8 Kohlenstoffatomen, Trifluormethyl oder Trifluormethoxy substituiert ist,

$R^4$ für Wasserstoff oder für geradkettiges oder verzweigtes Acyl mit bis zu 8 Kohlenstoffatomen oder Benzoyl steht,

und deren Salze.

2. Chinolin-2-yl-methoxybenzylhydroxyharnstoffe nach Anspruch 1

worin

A, B, D, E, G, L und M gleich oder verschieden sind und
für Wasserstoff, Hydroxy, Fluor, Chlor, Brom, Carboxy, Nitro, Trifluormethyl, Trifluormethoxy oder
für geradkettiges oder verzweigtes Alkyl oder Alkoxy mit jeweils bis zu 6 Kohlenstoffatomen stehen, oder

für Phenyl stehen, das gegebenenfalls durch Fluor, Chlor, Brom, Hydroxy, Nitro oder Cyano substituiert ist,

$R^1$ für Cyclobutyl, Cyclopentyl, Cyclohexyl, Cycloheptyl oder Cyclooctyl steht,
für geradkettiges oder verzweigtes Alkyl mit bis zu 6 Kohlenstoffatomen steht,

$R^2$ und $R^3$ gleich oder verschieden sind und
für Wasserstoff oder für geradkettiges oder verzweigtes Alkyl mit bis zu 6 Kohlenstoffatomen stehen,

oder

$R^2$ für Wasserstoff steht

und

$R^3$ für eine Gruppe der Formel $-SO_2R^5$ steht,

worin

$R^5$ geradkettiges oder verzweigtes Alkyl mit bis zu 6 Kohlenstoffatomen bedeutet, das gegebenenfalls durch Fluor, Chlor, Nitro, Cyano, Hydroxy oder durch Phenyl substituiert ist, oder Phenyl bedeutet, das gegebenenfalls bis zu 2-fach gleich oder verschieden durch Fluor, Chlor, Brom, Nitro, Cyano, Hydroxy, geradkettiges oder verzweigtes Alkyl, Alkylthio oder Alkoxy mit bis zu 6 Kohlenstoffatomen, Trifluormethyl oder Trifluormethoxy substituiert ist,

$R^4$ für Wasserstoff oder für geradkettiges oder verzweigtes Acyl mit bis zu 6 Kohlenstoffatomen oder Benzoyl steht

und deren Salze.

**3.** Chinolin-2-yl-methoxybenzylhydroxyharnstoffe nach Anspruch 1

in welchen

| | |
|---|---|
| A, B, D, E, G, L und M | gleich oder verschieden sind und für Wasserstoff, Hydroxy, Fluor, Chlor, Brom oder geradkettiges oder verzweigtes Alkyl mit bis zu 4 Kohlenstoffatomen stehen, |
| $R^1$ | für Cyclopentyl, Cyclohexyl oder Cycloheptyl steht, für geradkettiges oder verzweigtes Alkyl mit bis zu 4 Kohlenstoffatomen steht, |
| $R^2$ und $R^3$ | gleich oder verschieden sind und für Wasserstoff oder geradkettiges oder verzweigtes Alkyl mit bis zu 4 Kohlenstoffatomen stehen, oder |
| $R^2$ | für Wasserstoff steht, |

und

$R^3$ für eine Gruppe der Formel $-SO_2R^5$ steht,

worin

$R^5$ geradkettiges oder verzweigtes Alkyl mit bis zu 4 Kohlenstoffatomen bedeutet, das gegebenenfalls durch Fluor, Chlor oder Phenyl substituiert ist, oder Phenyl bedeutet, das gegebenenfalls durch Fluor, Chlor, Brom oder durch geradkettiges oder verzweigtes Alkyl mit bis zu 4 Kohlenstoffatomen substituiert ist,

$R^4$ für Wasserstoff oder für geradkettiges oder verzweigtes Acyl mit bis zu 4 Kohlenstoffatomen oder Benzoyl steht

und deren Salze.

**4.** Chinolin-2-yl-methoxybenzylhydroxyharnstoffe nach Anspruch 1

worin der Rest $-HC(R^1)N(OR^4)(CO-NR^2R^3)$ in 4-Stellung zum Chinolylmethoxyrest steht.

**5.** Chinolin-2-yl-methoxybenzylhydroxyharnstoffe nach Anspruch 1 zur Bekämpfung von Krankheiten.

**6.** Verfahren zur Herstellung von Chinolin-2-yl-methoxybenzylharnstoffen der allgemeinen Formel

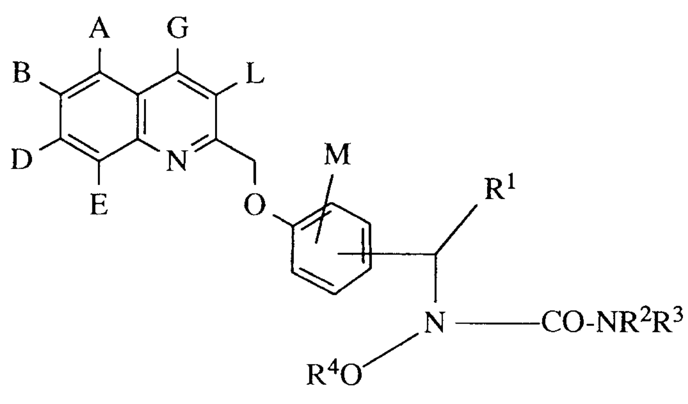

(I),

in welcher

| | |
|---|---|
| A, B, D, E, G, L und M | gleich oder verschieden sind und für Wasserstoff, Hydroxy, Halogen, Cyano, Carboxy, Nitro, Trifluormethyl, Trifluormethoxy oder für geradkettiges oder verzweigtes Alkyl oder Alkoxy mit jeweils bis zu 8 Kohlenstoffatomen stehen, oder für Aryl mit 6 bis 10 Kohlenstoffatomen stehen, das gegebenenfalls durch Halogen, Hydroxy, Nitro oder Cyano substituiert ist, |
| $R^1$ | für Cycloalkyl oder-alkenyl mit 3 bis 12 Kohlenstoffatomen steht, für geradkettiges oder verzweigtes Alkyl mit bis zu 8 Kohlenstoffatomen steht, |

$R^2$ und $R^3$ gleich oder verschieden sind und für Wasserstoff, geradkettiges oder verzweigtes Alkyl mit bis zu 8 Kohlenstoffatomen oder für Phenyl oder Benzyl stehen, oder

$R^2$ für Wasserstoff steht

und

$R^3$ für eine Gruppe der Formel $-SO_2R^5$ steht,

worin

$R^5$ geradkettiges oder verzweigtes Alkyl mit bis zu 8 Kohlenstoffatomen bedeutet, das gegebenenfalls durch Halogen, Nitro, Cyano, Hydroxy, Trifluormethyl oder durch Aryl mit 6 bis 10 Kohlenstoffatomen substituiert ist,

oder

Aryl mit 6 bis 10 Kohlenstoffatomen bedeutet, das bis zu 3-fach gleich oder verschieden durch Halogen, Nitro, Cyano, Hydroxy, geradkettiges oder verzweigtes Alkyl, Alkylthio oder Alkoxy mit bis zu 8 Kohlenstoffatomen, Trifluormethyl oder Trifluormethoxy substituiert ist,

$R^4$ für Wasserstoff oder für geradkettiges oder verzweigtes Acyl mit bis zu 8 Kohlenstoffatomen oder Benzoyl steht,

und deren Salze,

dadurch gekennzeichnet, daß man

Verbindungen der allgemeinen Formel

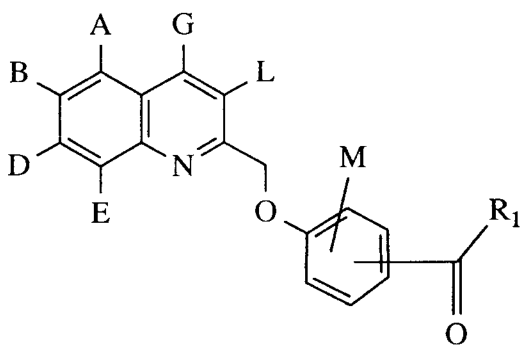

(II)

in welcher

A, B, D, E, G, L, M und $R^1$ die oben angegebene Bedeutung haben,

zunächst in inerten Lösemitteln, gegebenenfalls in Anwesenheit einer Base mit Hydroxylaminhydrochlorid zu den Ketoximen der allgemeinen Formel (III)

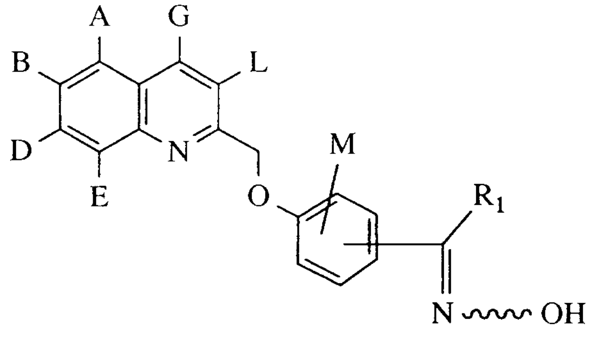

(III)

in welcher

A, B, D, E, G, L, M und $R^1$ die oben angegebene Bedeutung haben,

umsetzt, anschließend in inerten Lösemitteln, gegebenenfalls in Anwesenheit von Säuren, mit Reduktionsmitteln in die Hydroxylamine der allgemeinen Formel (IV)

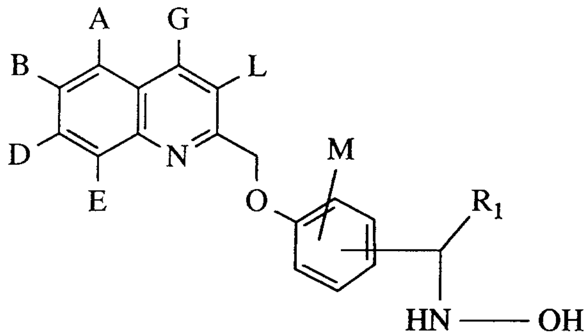

(IV)

in welcher

A, B, D, E, G, L, M und $R^1$ die oben angegebene Bedeutung haben,

überführt, und in einem letzten Schritt im Fall, daß $R^2$ und $R^3$ = H mit ($C_1$-$C_6$)-Trialkylsilylisocyanaten, vorzugsweise mit Trimethylsilylisocyanat, und im Fall, daß $R^2$ = H und $R^3 \neq$ H mit Isocyanaten der allgemeinen Formel (V)

$T\text{-}N=C=O$ (V)

in welcher

T für ($C_1$-$C_8$)-Alkyl, Phenyl oder für den Rest $-SO_2R^5$ steht,

worin

$R^5$ die oben angegebene Bedeutung hat,

umsetzt,

oder

Hydroxylamine der allgemeinen Formel (IV) zunächst mit gasförmiger HCl und anschließend mit Phosgen in die Verbindungen der allgemeinen Formel (VI)

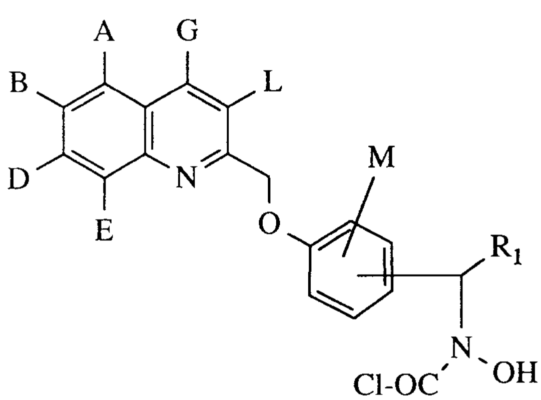

(VI)

in welcher

A, B, D, E, G, L, M und $R^1$ die oben angegebene Bedeutung haben,

in inerten Lösemitteln überführt und nachfolgend, gegebenenfalls in inerten Lösemitteln, mit Aminen der allgemeinen Formel (VII)

$HNR^2R^3$ (VII)

in welcher

$R^2$ und $R^3$ gleich oder verschieden sind und die oben angegebene Bedeutung haben,

umsetzt und im Fall, daß $R^4 \neq H$, Verbindungen der allgemeinen Formel (Ia)

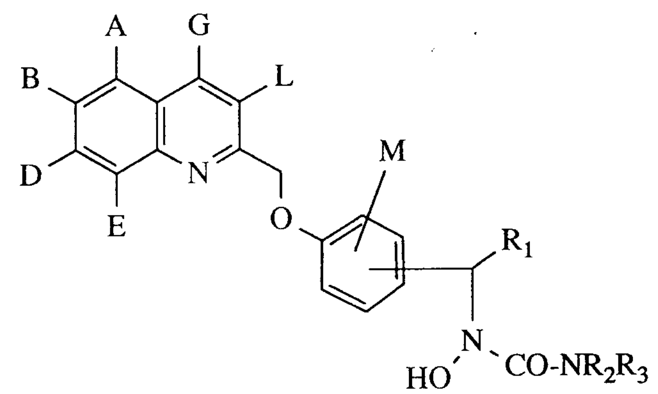

(Ia)

in welcher

A, B, D, E, G, L, M, $R^1$, $R^2$ und $R^3$ die oben angegebene Bedeutung haben,

mit üblichen Acylierungsmitteln, vorzugsweise mit Säurehalogeniden der allgemeinen Formel (VIII)

$R^{4'}$-CO-X (VIII)

in welcher

$R^{4'}$ die oben angegebene Bedeutung von $R^4$ hat, aber nicht für Wasserstoff steht

und

X für eine in Acylierungsmitteln übliche "leaving group", vorzugsweise für Chlor steht,

in inerten Lösemitteln, gegebenenfalls in Anwesenheit einer Base, acyliert.

**7.** Arzneimittel enthaltend mindestens ein Chinolin-2-yl-methoxybenzylhydroxyharnstoff nach Anspruch 1.

**8.** Verwendung von Chinolin-2-yl-methoxybenzylhydroxyharnstoffen nach Anspruch 1 zur Herstellung von Arzneimitteln.

**9.** Verwendung von Chinolin-2-yl-methoxybenzylhydroxyharnstoffen nach Anspruch 1 zur Herstellung von Lipoxygenasehemmern.

**10.** Verwendung von Chinolin-2-yl-methoxybenzylhydroxyharnstoffen nach Ansprach 1 zur Bekämpfung von Krankheiten.

**11.** Verbindungen der allgemeinen Formel

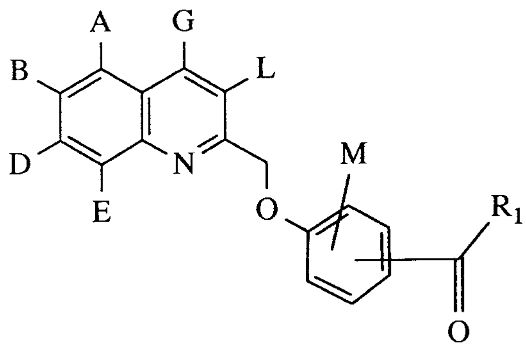

(II)

in welcher

A, B, D, E, G, L und M gleich oder verschieden sind und

für Wasserstoff, Hydroxy, Halogen, Cyano, Carboxy, Nitro, Trifluormethyl, Trifluormethoxy oder

für geradkettiges oder verzweigtes Alkyl oder Alkoxy mit jeweils bis zu 8 Kohlenstoffatomen stehen, oder für Aryl mit 6 bis 10 Kohlenstoffatomen stehen, das gegebenenfalls durch Halogen, Hydroxy, Nitro oder Cyano substituiert ist,

und

$R^1$ für Cycloalkyl oder-alkenyl mit 3 bis 12 Kohlenstoffatomen steht.

Europäisches Patentamt

# EUROPÄISCHER RECHERCHENBERICHT

Nummer der Anmeldung

EP 92 11 2359

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 414 019 (BAYER AG)<br>* Ansprüche *<br>--- | 1,7,9 | C07D215/14<br>A61K31/47 |
| D,A | EP-A-0 416 609 (ABBOTT LABORATORIES)<br>* Ansprüche *<br>--- | 1,7,9 | |
| D,A | WO-A-8 904 299 (ABBOTT LABORATORIES)<br>* Ansprüche *<br>----- | 1,7,9 | |

RECHERCHIERTE SACHGEBIETE (Int. Cl.5)

C07D

Der vorliegende Recherchenbericht wurde für alle Patentansprüche erstellt

| Recherchenort | Abschlußdatum der Recherche | Prüfer |
|---|---|---|
| DEN HAAG | 09 OKTOBER 1992 | VAN BIJLEN H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)